(19) Europäisches Patentamt
European Patent Office
Office européen des brevets

(11) EP 1 723 959 A1

(12) EUROPEAN PATENT APPLICATION
published in accordance with Art. 158(3) EPC

(43) Date of publication:
**22.11.2006 Bulletin 2006/47**

(21) Application number: **05709457.5**

(22) Date of filing: **28.01.2005**

(51) Int Cl.:
***A61K 35/16*** *(2006.01)* ***A61L 27/00*** *(2006.01)*
***A61P 1/02*** *(2006.01)* ***A61P 17/02*** *(2006.01)*
***A61P 19/00*** *(2006.01)* ***A61P 25/00*** *(2006.01)*
***A61P 41/00*** *(2006.01)* ***A61P 43/00*** *(2006.01)*
***A61J 1/00*** *(2006.01)*

(86) International application number:
**PCT/JP2005/001231**

(87) International publication number:
**WO 2005/072753 (11.08.2005 Gazette 2005/32)**

(84) Designated Contracting States:
**AT BE BG CH CY CZ DE DK EE ES FI FR GB GR HU IE IS IT LI LT LU MC NL PL PT RO SE SI SK TR**

(30) Priority: **30.01.2004 JP 2004024815**

(71) Applicants:
- **Sumida, Emi**
  **Tokyo 113-0023 (JP)**
- **TORAY INDUSTRIES, INC.**
  **Tokyo 103-8666 (JP)**

(72) Inventors:
- **SUMIDA, Emi**
  **1540002 (JP)**
- **KASUGAI, Shohei,**
  **2-23-2, Nagahama, Kanazawa-ku,**
  **Kanagawa 2360011 (JP)**
- **AKIYOSHI, Kazunari**
  **1020081 (JP)**
- **IWASAKI, Yasuhiko**
  **2060013 (JP)**

(74) Representative: **Krauss, Jan**
**Forrester & Boehmert,**
**Pettenkoferstrasse 20-22**
**80336 München (DE)**

# (54) COMPOSITION COMPRISING PLATELET-RICH PLASMA

(57) The present invention provides a platelet-rich plasma with high activities in an easy and economical manner.
The platelet-rich plasma is obtained by aggregating and precipitating erythrocytes from collected whole blood selectively and facilitatively without employing centrifugation. For example, the plasma is obtained by the addition of a polymer having a residue of an organic phosphate compound. Specifically, the polymer having a residue of an organic phosphate compound can be added to whole blood and allowed to stand for a specific time, thereby to selectively and facilitatively aggregate and precipitate erythrocytes to give a supernatant. The supernatant contains much platelets which exist in their extremely intact state similar as in a living body and simultaneously provides a platelet-rich plasma with high activity comprising much plasma proteins such as fibrinogen and leukocytes.

EP 1 723 959 A1

**Description**

Technical Field

The present invention relates to platelet-rich plasma to be used in the field of medical treatment and a method for the preparation thereof.
The present application claims the priority of Japanese Patent Application No. 2004-024815, which is incorporated herein by reference.

Background Art

Currently, medicine and engineering are making a great progress in the field of regeneration of lost tissue. However, recently, the use of, for example, a blood product for medical use or a physiologically active substance produced from an animal (in particular, cow), which is contaminated with viruses ,prion and so on, are often cause infection, leading to a safety threatening events as a social problem. From this fact, a growing interest is focused on a safety concerning all medical treatments.
Further, for routine clinical applications, besides safety, reliability and convenience in operation are also important.
From such background, a fibrin formulation, which has an effect acknowledged as an enhancer for hemostasis and wound-healing after surgery, has been developed since early times to attract lots of attention on a therapeutic method for facilitating to heal a wound using self-blood components. For example, because an activated platelet secretes a substance which induces cell migration and differentiation at an early stage of wound healing, a plasma comprising the platelets concentrated from self-blood (platelet-rich plasma, hereinafter simply referred to as "PRP") is prepared before surgery and activated to apply onto an operation site, thereby to facilitate the therapy. Many clinicians have reported that this method has succeeded in facilitating wound healing.
This method is a very safe method using self-blood and provides a reliable performance to some extent, but it has problems such as complexity to work, riskiness, laboriousness, necessity for skilled staffs, expensive devices to purchase and increasing cost for maintenance.
Methods for preparing platelet-rich plasma, as well as methods and apparatus for separating blood into its components and the like have already been described in many reports (patent document No.1). Platelet-rich plasma for clinical testing can be obtained from a supernatant which is prepared by collecting blood from the median vein of forearm, adding each 4.5 mL of the whole blood into plastic tubes containing 1. 5 mL of 3.1 w/v% sodium citrate, tumbling to mix, and then centrifuging by 500 g for 15 minutes at 22°C (non-patent document No. 1). Conventional centrifugation can precipitate erythrocytes, but needs a complicated work and can not be exempted from damaging hemocytes such as platelets and plasma proteins.
In the following, platelet-rich plasma obtained by a conventional method is referred to as "PRP" for convenience to differentiate from the platelet-rich plasma of the present invention.
To obtain leukocyte-rich plasma (LRP), a method for adding both a rouleau formation aggregating agent and an enhancer to whole blood to separate erythrocyte cells from the whole blood is disclosed (patent document No. 2). Here, as the rouleau formation aggregating agent, for example, dextran, Hespan, Pentaspan and Ficoll are mentioned, and as the enhancer, oxalate, malonate, mannitol and sucrose are mentioned.
Platelet-rich plasma used as an enhancer for hemostasis or wound-healing after surgery is desired to comprise not only a platelet component but also much blood components including plasma proteins such as fibrinogen and leukocytes in the most same possible state as they are present in a living body. There is desired a platelet-rich plasma having so high an activity.
Even though PRP is obtained by centrifugation or precipitation of hemocytes from blood, the PRP must contain not only platelets at least with their activity already enhanced but also a coagulation reaction inducer such as thrombin to use as a tissue and/or organ repair enhancer.

[patent document No. 1] JP H5-203639-A
[patent document No. 2] JP H8-510322
[non-patent document No.1] "Outline of Clinical Laboratory Testing", 31st ed., pp. 400

Disclosure of the Invention

(Problems to be solved)

An object of the present invention is to provide easily and inexpensively a platelet-rich plasma which does not always need a coagulation reaction inducer such as thrombin to add and has an effectively high activity to use as a

tissue and/or organ repair enhancer.

(Means to Solve the Problems)

The present inventors studied strenuously and, as a result, found that a polymer having a residue of an organic phosphate compound is added to a whole blood to aggregate and precipitate erythrocytes selectively and facilitatively, allowing provision of a platelet-rich plasma which comprises intact platelets similar to those in a living body and can be used as a tissue and/or organ repair enhancer free from a coagulation reaction inducer such as thrombin. The finding completes the present invention.

Therefore, the present invention is composed of the following:

1. A method for preparing platelet-rich plasma, comprising adding a polymer having a residue of an organic phosphate compound to blood.
2. A method for preparing platelet-rich plasma, comprising the step of adding a polymer having a residue of an organic phosphate compound to blood to precipitate erythrocytes selectively.
3. The method for preparing platelet-rich plasma according to the preceding clause 1 or 2, comprising, as the polymer having a residue of an organic phosphate compound, a compound having in its side chain a group represented by the Formula (I) below:

[Chem. 1]

Formula (I)

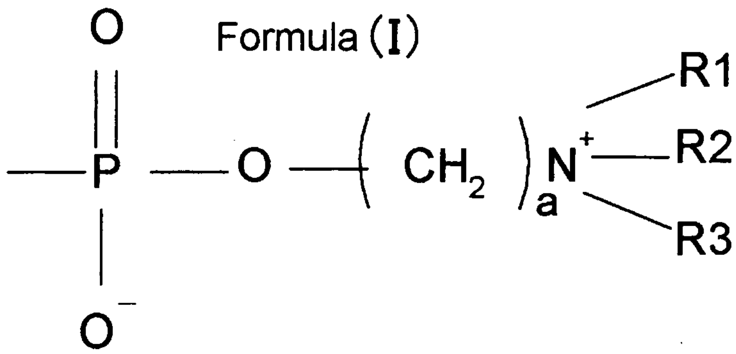

(wherein R1, R2 and R3 are each independently a straight or branched chain alkyl group having a carbon number of 1 to 8, and a is an integer of 1 to 12).

4. The method for preparing platelet-rich plasma according to the preceding clause 3, comprising, as a polymer having a residue of an organic phosphate compound, a compound represented by the Formula (II) below:

[Chem. 2]

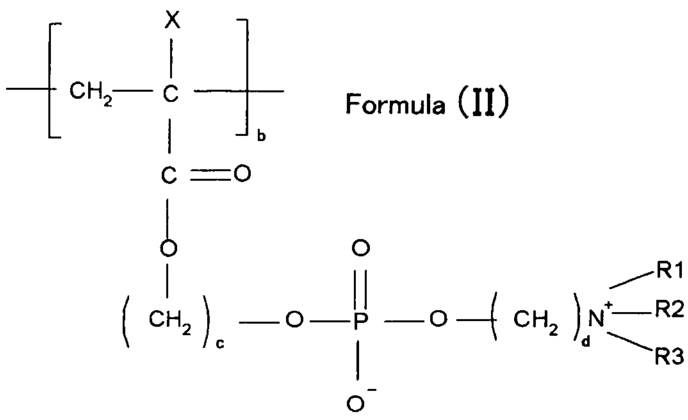

(wherein X is -H or $-CH_3$, R1, R2 and R3 are each independently a straight or branched chain alkyl group having a carbon number of 1 to 8, b is an integer of 1 to 4,000, c is an integer of 1 to 6, and d is an integer of 1 to 12).

5. The method for preparing platelet-rich plasma according to any one of the preceding clauses 1 to 4, further comprising, as a polymer having a residue of an organic phosphate compound, a compound represented by the Formula (III) below:

[Chem. 3]

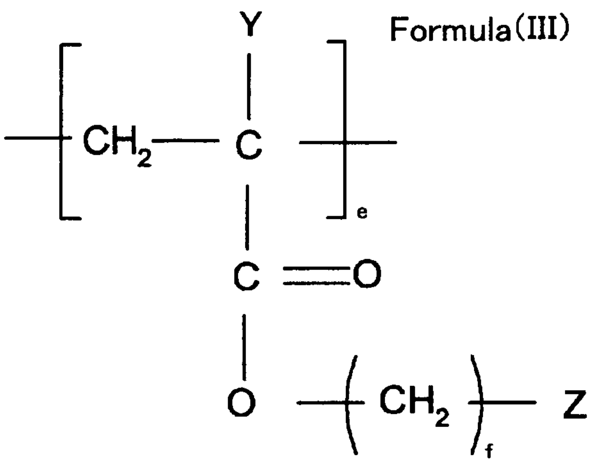

(wherein Y is -H or $-CH_3$, Z is -H, -OH, -COOH, -COONa, -COOK, $-SO_3H$, $-SO_3Na$, $-SO_3K$, $-NH_2$, -NHR1, -NR1R2, $-N^+R1R2R3OH^-$, $-N^+R1R2R3SO_3-$, $-N^+R1R2R3NO_3-$, $-N^+R1R2R3PO_3-$ [wherein R1, R2 and R3 are independently $-(CH_2)xH$, wherein x is an integer of 1 to 5], $-O-PO_3Na$, $-O-PO_3K$, $-O-PO_3H$, -OR4H, -COOR4H, -C=O-R4H, [wherein R4 is aliphatic hydrocarbon, wherein the number of hydrocarbon is 0 to 20] or -O-R5 [R5 is an aromatic hydrocarbon group], e is an integer of 1 to 4000, and f is an integer of 0 to 20).

6. The method for preparing platelet-rich plasma according to the preceding clause 4 or 5, comprising, as a polymer having a residue of an organic phosphate compound, a compound represented by the Formula (IV) below:

[Chem. 4]

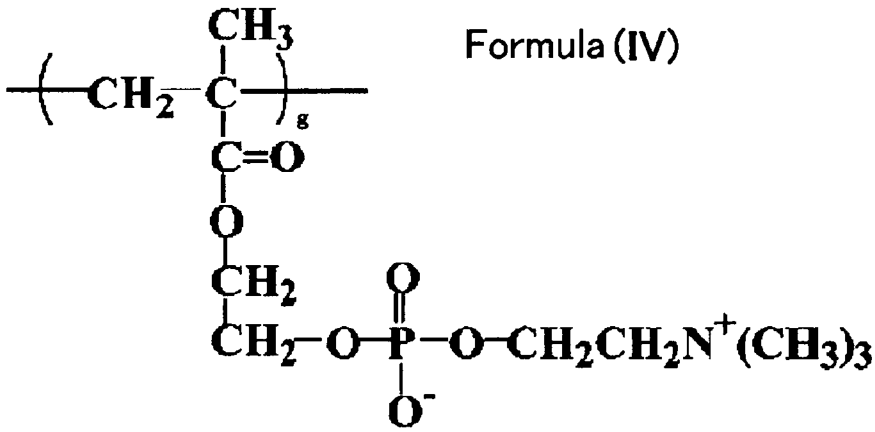

Formula (IV)

(wherein g is an integer of 1 to 4,000).

7. The method for preparing the platelet-rich plasma according to any one of the preceding clauses 3 to 6, comprising, as a polymer having a residue of an organic phosphate compound, a compound represented by any of the Formulae (V) to (VII) below:

[Chem. 5]

Formula (V)

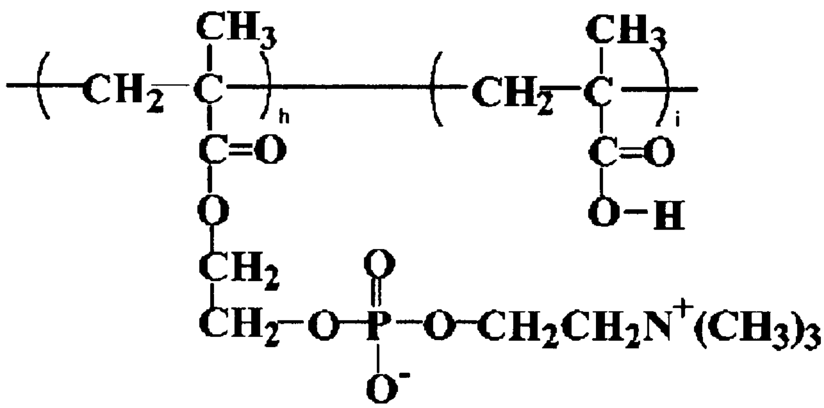

(wherein h and i are each independently an integer of 1 to 4,000),

[Chem. 6]

Formula (VI)

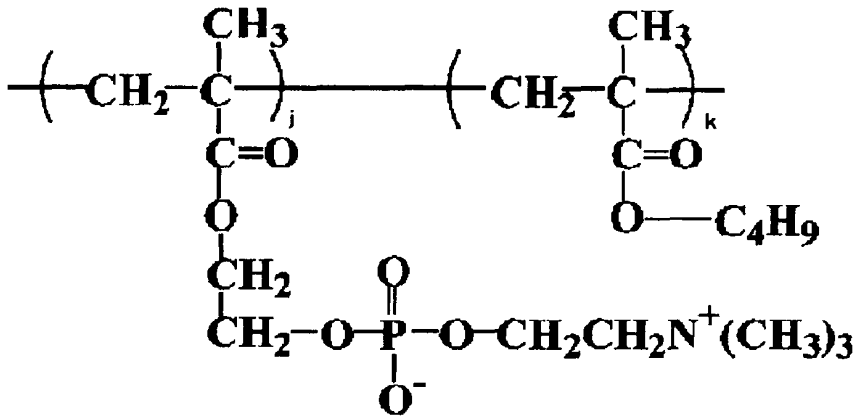

(wherein j and k are each independently an integer of 1 to 4,000), and

[Chem. 7]

Fromula (VII)

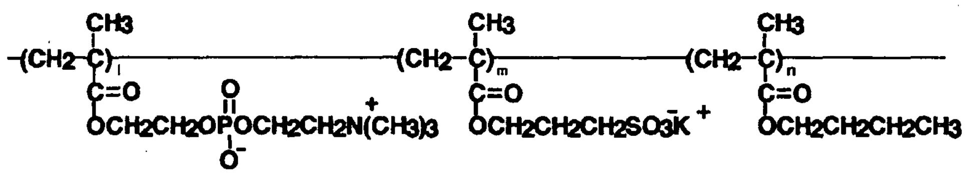

(wherein 1, m and n are each independently an integer of 1 to 4,000).

8. Platelet-rich plasma obtained by the method for preparing according to any one of the preceding clauses 1 to 7.

9. A tissue and/or organ repair enhancer comprising the platelet-rich plasma according to the preceding clause 8.

10. A tissue and/or organ repair enhancer, augmentation additive of bone surrounding dental implant, additive when implanting bone or artificial bone into a bone deficient part, wound healing enhancer, tissue healing enhancer after therapy or treatment for plastic and/or cosmetic purpose, skin disease therapeutic agent, skin ulcer therapeutic agent, nerve tissue repair agent and/or tissue repair agent after surgery comprising the platelet-rich plasma according to the preceding clause 8.

11. Any of the following therapeutic methods or treatment methods, comprising administering the platelet-rich plasma according to the preceding clause 8:

1) augmenting bone surrounding dental implant,
2) skin disease,
3) repairing tissue for plastic and/or cosmetic purpose,
4) repairing bone deficient part,
5) repairing nerve tissue, and
6) repairing tissue after surgery.

12. A polymer for preparing the platelet-rich plasma according to the preceding clause 8.

13. A reagent or a reagent kit for preparing platelet-rich plasma comprising the polymer according to the preceding clause 12.

14. A device for preparing the platelet-rich plasma according to the preceding clause 8.

(Effects of Invention)

A polymer having a residue of an organic phosphate compound of the present invention is added to a blood to aggregate and precipitate erythrocytes more selectively than other hemocytes, allowing provision of platelet-rich plasma comprising a platelet which is little damaged and exists in its extremely intact state. The platelet-rich plasma, therefore, can be used as an effective tissue and/or organ repair enhancer. Further, the platelet-rich plasma obtained by the present method can be used with a reduced amount of a coagulation reaction inducer such as thrombin or to be free from the inducer as the case may be.

Brief Description of the Drawings

Figure 1 shows the states of precipitated erythrocytes after the polymer was added to blood and allowed to stand for 20 minutes and 30 minutes. (Example 7)
Figure 2 shows platelet recovery rates of both platelet-rich plasma of the present invention and the PRP obtained by centrifugation of a conventional method. (Example 8)
Figure 3 shows amounts of fibrinogen contained in the platelet-rich plasma of the present invention and the PRP obtained by centrifugation of a conventional method. (Example 8)
Figure 4 shows activated-platelet rates (CD62P) in the platelet-rich plasma of the present invention and the PRP obtained by centrifugation of a conventional method. (Example 8)

Description of the Preferred Embodiment

Platelets adhere and aggregate to damaged tissue to form thrombus, and play an extremely important role for hemostasis and further for storing and releasing substances which induce other cells to migrate and differentiate. Fibrin is produced by the action of thrombin on fibrinogen in plasma and involved in the final stage of blood coagulation. Further, fibrin is important as a scaffold for cells to infiltrate and differentiate in order to repair tissue. Further, it is known that leukocytes serve to prevent bacteria and harmful microorganisms from invading and have an immune function and a biocidal function, and in particular monocytes/macrophages among them play a key role in tissue repair.

Platelets rapidly lose its function as time passes after stimulation or blood collection. Thereby, any other approach than centrifugation which little stimulates platelets and can separate quickly could not provide platelets holding functions to some extent. It is known that whole blood is allowed to stand to precipitate erythrocytes gradually, but that the rate is usually so little that no other blood can be left for several hours or more to provide a platelet-containing plasma than a sick blood such as a blood in a high level inflammatory reaction.

The method for preparing platelet-rich plasma of the present invention can precipitate erythrocytes selectively and facilitatively from whole blood to give a supernatant which contains components essential for tissue repair not only platelets but also leukocytes and fibrinogen in their almost damage-free states with their respective activities held. Further, the platelet-rich plasma obtained by the present invention comprises platelets which are not almost activated to take the most possible intact state. The platelets in the most possible intact state have characters such as high hemostatic and coagulation effects and also maintain the high capacity to release a growth factor, so that platelet-rich plasma comprising such platelets has an excellent quality.

By the present invention, erythrocytes can be selectively and facilitatively aggregated and precipitated. 80% or more, preferably 90% or more of erythrocytes contained in whole blood can be precipitated by aggregation within three hours, preferably within two hours, more preferably within an hour, and even more preferably within 30 minutes after starting the treatment, thereby to provide platelet-rich plasma of at least 15% or more of total blood volume.

Whole blood for use in the preparation of platelet-rich plasma of the present invention may be obtained according to the usual method for blood collection in human or animals other than human. Further, since the collected whole blood is left intact to coagulate, an anticoagulant is desired to add early after blood collection. As the anticoagulant, any may be used as long as it is commonly used and shows no toxicity to a living body, thus for example, those generally used such as sodium citrate, anticoagulant acid-citric-dextrose (ACD), EDTA, heparin, low molecular heparin, Futhan, hirudin and argatroban may be used.

The amount of blood to be treated for preparing platelet-rich plasma of the present invention is not limited in particular and varied by usage, thus the adequate amount can be selected depending on the purpose or amount of use. The method of the present invention is applicable, for example, to self-blood as well as bloods collected from blood donors. As described above, the present invention is a method to conveniently obtain platelets with a high activity, thus

it can also be used to prepare platelet formulations.

(A polymer having a residue of an organic phosphate compound)

To selectively and facilitatively aggregate and precipitate erythrocytes from whole blood, a polymer having a residue of an organic phosphate compound can be added to whole blood. As the polymer having a residue of an organic phosphate compound, a compound comprising, as its side chain, a group represented by the Formula (I) below can be included in the constituent units:

[Chem. 8]

Formula (I)

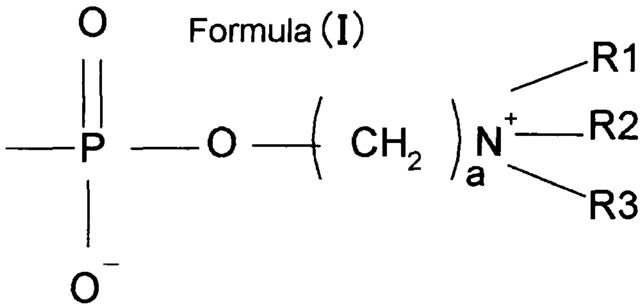

(wherein R1, R2 and R3 are each independently a straight or branched chain alkyl group having a carbon number of 1 to 8, and a is an integer of 1 to 12).

As the polymer having a residue of an organic phosphate compound, specifically a compound represented by the Formula (II) below can be included:

[Chem. 9]

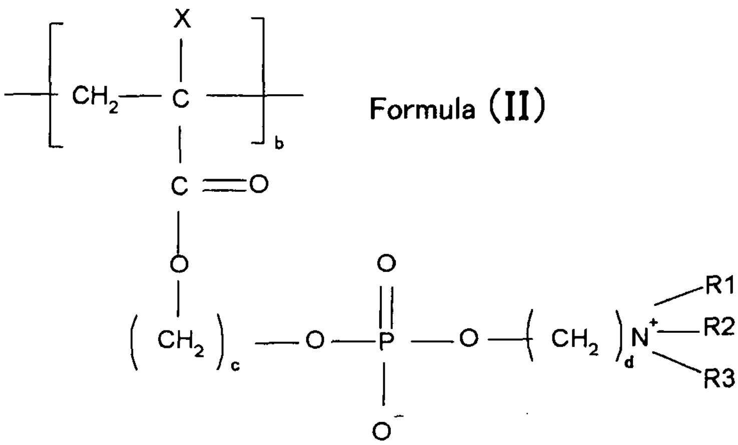

Formula (II)

(wherein X is -H or -$CH_3$, R1, R2 and R3 are each independently a straight or branched chain alkyl group having a carbon number of 1 to 8, b is an integer of 1 to 4000, c is an integer of 1 to 6, d is an integer of 1 to 12).

Further, the polymer for use in the present invention may be a homopolymer comprising only one compound selected from Formula (II) as the constituent unit, or may be a heteropolymer comprising two or more compounds selected from Formula (II) as the constituent unit. Further, the polymer may be a heteropolymer comprising the other constituent units. Specifically, the polymer may include a methacrylate compound represented by Formula (III):

[Chem. 10]

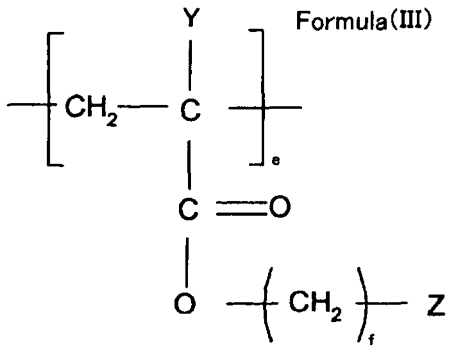

Formula (III)

(wherein Y is -H or $-CH_3$, Z is -H, -OH, -COOH, -COONa, -COOK, $-SO_3H$, $-SO_3Na$, $-SO_3K$, $-NH_2$, -NHR1, -NR1R2, $-N^+R1R2R3OH^-$, $-N^+R1R2R3SO_3-$, $-N^+R1R2R3NO_3-$, $-N^+R1R2R3PO_3-$ [wherein R1, R2 and R3 are independently $-(CH_2)xH$, wherein x is an integer of 1 to 5], $-O-PO_3Na$, $-O-PO_3K$, $-O-PO_3H$, -OR4H, -COOR4H, -C=O-R4H, [wherein R4 is aliphatic hydrocarbon, wherein the number of hydrocarbon is 0 to 20] or -O-R5 [R5 is an aromatic hydrocarbon group], e is an integer of 1 to 4000, and f is an integer of 0 to 20).

As the polymer for use in the present invention, specifically a methacrylate polymer can be mentioned and, for example, 2-methacryloyl oxyethyl phosphorylcholine (MPC) represented by Formula (IV) below or derivatives thereof may be included:

[Chem. 11]

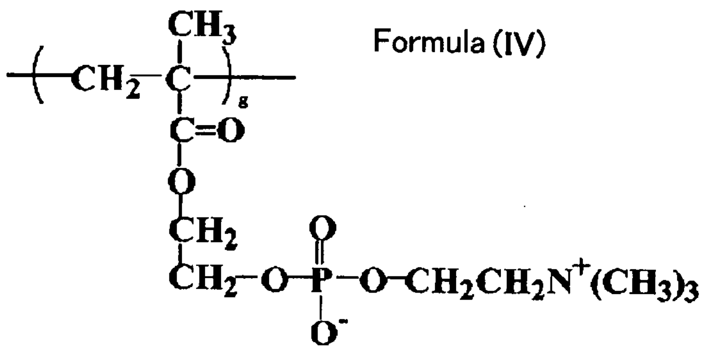

Formula (IV)

(wherein g is an integer of 1 to 4000).

As an example of heteropolymer for use in the present invention, compounds represented by the Formulae (V) and/or (VI) below can be included:

[Chem. 12]

Formula (V)

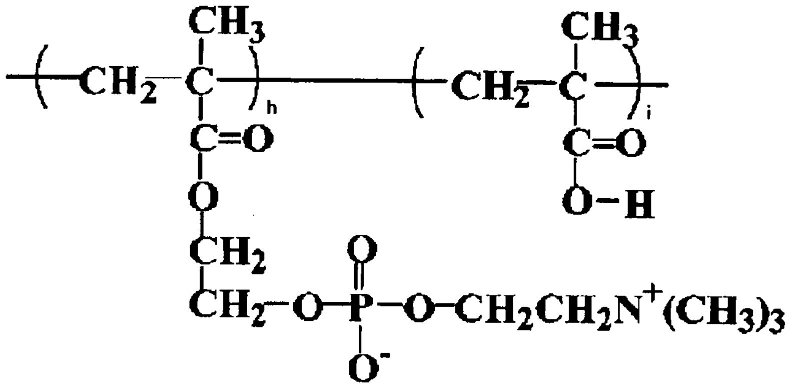

[Chem. 13]

Formula (VI)

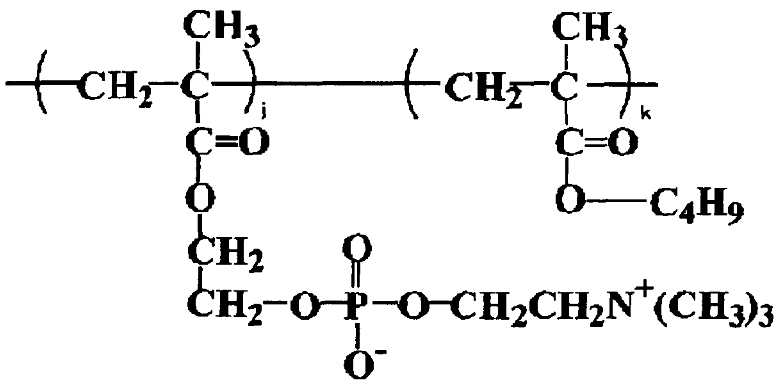

(wherein h, i, j and k are each independently an integer of 1 to 4000). For example, two kinds of constituent compounds represented in Formulae (V) and (VI) can have an appropriately selected ratio and, for example, 2-methacryloyl oxyethyl phosphorylcholine (MPC) and a methacrylate compound can have an appropriately selected constituent ratio such as 7:3, 8:2 and 9:1.

Further, as an example of heteropolymer for use in the present invention, a compound represented by the Formula (VII) below can be included:

[Chem. 14]

Fromula (VII)

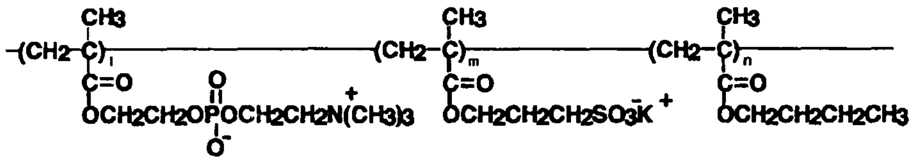

(wherein 1, m and n are each independently an integer of 1 to 4000), and the represented three constituent compounds can have an appropriately selected ratio. 2-methacryloyl oxyethyl phosphorylcholine (MPC), Potassium 3-Methacryloyloxypropyl sulfonate (PMPS) and n-butyl methacrylate (BMA), which are contained in PMSB of Formula (VII), can have an appropriately selected ratio such as 25:20:55, 45:5:50 and 41:4:55. Meanwhile, for the purposes herein, PMSB25 means the polymer wherein the constituent ratio of MPC:PMPS:BMA is 25:20:55 and PMSB45 means the polymer wherein the constituent ratio of MPC:PMPS:BMA is 45:5:50.

The average molecular weight of polymer applicable to the present invention is preferably in the range from 1,000 to 5,000,000, and more preferably from 1,000 to 1,000,000.

(The method for preparing platelet-rich plasma)

To prepare the platelet-rich plasma of the present invention, into 1. 5 mL of blood containing an anticoagulant, 0.0015 to 150 mg of, preferably 0.15 to 45 mg of, and more preferably 1.5 to 4.5 mg of the above described polymer having a residue of an organic phosphate compound can be added. They correspond to their respective concentrations of approximately from 0.0001 to 10 w/v%, preferably from 0.01 to 3 w/v%, and more preferably from 0.1 to 0.3 w/v%. For preparing a larger amount of platelet-rich plasma, the blood volume should be increased and the polymer having a residue of an organic phosphate compound can be added to have the same rate as those described above. The above described polymer having a residue of an organic phosphate compound can be previously added in a container for holding collected blood, or directly in an injection syringe for blood collection.

The polymer of an amount selected from the above volume range is added to blood containing an anticoagulant, and mixed gently to diffuse the polymer uniformly in blood, and then allowed to stand. Erythrocytes can be observed to precipitate selectively and facilitatively, thereby to give a supernatant, from which platelet-rich plasma can be obtained to comprise not only platelets but also a plasma component and a blood components such as leukocytes. Precipitation of erythrocytes can be observed in 3 hours at the longest, preferably within 2 hours, and as early as about 10 minutes after starting the treatment, so that the objective platelet-rich plasma of the present invention can be obtained within about 30 to 40 minutes.

Further, if erythrocytes are not sufficiently separated, or a shorter time of separation is needed, conventional weak centrifugation can further be conducted to such an extent that no platelets may be aggregated. Specifically, centrifugation by 142 G for 5 minutes or less can be applied.

(The use of platelet-rich plasma of the present invention)

The platelet-rich plasma prepared by the method of the present invention can be applied for any tissue or organ as a healing enhancer such as a wound healing agent, an augmentation additive of bone surrounding dental implant, an additive for implanting bone or artificial bone into a bone deficient part, a wound healing enhancer, a skin disease therapeutic agent, a skin ulcer therapeutic agent, a tissue healing enhancer after therapy or treatment for plastic or cosmetic purpose, a tissue repair agent after surgery, a tissue repair agent after surgery at an orthopedic operation site and a nerve tissue repair agent. Thus, with the platelet-rich plasma of the present invention, those disorders described above and damages in skins or tissues can be treated.

The platelet-rich plasma of the present invention can be applied to human and mammals other than human. As an example of mammals, in particular, animals on the ground may be mentioned, and usually it can be applied to those kept as a pet such as dogs, cats and hamsters. Further, it can be applied to animals employed in sports such as racehorses and bulls for fight.

The platelet-rich plasma prepared by the method of the present invention from self-blood can be used for the purpose of therapy or treatment described above. Further, platelet-rich plasma, which is derived from the other blood than self-blood, can of course be used as long as the other blood is applicable in blood type.
For use, specifically, any administration methods are applicable, and, for example, a required amount of the platelet-rich plasma of the present invention can be applied or injected to a wound site.

(Preparative reagent, device and kit)

The platelet-rich plasma of the present invention is characterized in that; as described above, it can be prepared from self-blood conveniently and easily to be effective for therapy and the like. In order to achieve the convenient and easy preparation, a polymer having a residue of an organic phosphate compound for use in the preparation method of the present invention is stored as a reagent.
Specifically, a reagent in which various polymers explained in the section of "polymer having a residue of an organic phosphate compound" described above are filled in an appropriate container such as vial and ampoule, a plurality of these reagents, or a reagent kit comprising further a dissolution solution is also included in the present invention. Further, as a device for use in the preparation method of the present invention, specifically syringes and tubes for blood collection, and also sterilized containers or test tubes made of, for example, plastic, which are used to add a polymer having a residue of an organic phosphate compound to collected blood, may be mentioned. As the kit for preparing the platelet-rich plasma of the present invention, a kit which selects a reagent and a device selected from reagents and devices shown above to assemble may be mentioned. The present kit can be used to prepare the platelet-rich plasma of the present invention easily, for example, at the bedside where blood is collected.
Further, syringes and containers for use in collecting blood, in which the polymer having a residue of an organic phosphate compound of the present invention has been filled in advance, are preferably used. A syringe or a container described above filled with the polymer having a residue of an organic phosphate compound is particularly preferably used, wherein the polymer has been dissolved in sodium citrate or ACD which is broadly used as an anticoagulant.
The present invention covers the platelet-rich plasma prepared by the method described above and a specific polymer in the present invention for use in preparing platelet-rich plasma.

Example

In the following, the present invention will be explained in more detail with Examples and Experimental Examples, but the present invention will not be limited to them.

Example 1

This Example aims to confirm effects of various monomers or polymers added to whole blood (peripheral blood) to precipitate erythrocytes.
Seven samples were prepared by the following method. Anticoagulant acid-citrate-dextrose (hereinafter "ACD", from Shigma) was added to prepare a 3.13 w/v% ACD solution as an anticoagulant. 1.35 mL of blood was added to 0.15 mL of the ACD solution to prepare a whole blood sample (control) having a total of 1.5 mL.
In this Example, for Sample 2, a heteropolymer Poly(MPC-co-BMA) (PMB (8:2)) (molecular weight of 100,000) comprising MPC and BMA in a ratio of 8:2 as described by formula VI was used, while for Sample 3, a homopolymer (molecular weight of 100,000)composed of only MPC as a constituent unit as described by formula IV was used. As a polyglutamic acid, the one from Shigma (molecular weight of 53,785) was used.
Sample 1: Control
Sample 2: 3 mg of PMB (8:2) was dissolved in 0.15 mL of the ACD solution, and then mixed with 1.35 mL of blood.
Sample 3: 3 mg of PMPC was dissolved in 0.15 mL of the ACD solution, and then mixed with 1.35 mL of blood.
Sample 4: 0.1 mg of polyglutamic acid was dissolved in 0.15 mL of the ACD solution, and then mixed with 1.35 mL of blood.
Sample 5: 0.5 mg of polyglutamic acid was dissolved in 0.15 mL of the ACD solution, and then mixed with 1.35 mL of blood.
Sample 6: 0.7 mg of polyglutamic acid was dissolved in 0.15 mL of the ACD solution, and then mixed with 1.35 mL of blood.
Sample 7: 3 mg of polyglutamic acid was dissolved in 0.15 mL of the ACD solution, and then mixed with 1.35 mL of blood.

1) Precipitation of erythrocytes

The system of Sample 4 with polyglutamic acid added was almost equal to the systems of Samples 2 and 3 with the MPC-containing polymers added in Precipitation of erythrocytes after the samples were allowed to stand for 30 minutes.

2) Platelet count

Leukocytes (WBC), erythrocytes (RBC), and platelets (PLT) contained in the supernatants after the samples were allowed to stand for 30 minutes were counted. Counting was conducted with an automatic hemocyte counter (Celltacα (MEC-6318)) supplied by Nihon Kohden co..
As a result, the supernatant obtained from Sample 3 showed a high value for leukocyte and platelet counts, like the supernatant obtained from Sample 4 (Table 1).

[Table 1]

| | **Sample1** | **Sample2** | **Sample3** | **Sample4** | **Sample5** | **Sample6** | **Sample7** |
|---|---|---|---|---|---|---|---|
| **WBC** | **65** | **124** | **140** | **140** | **100** | **97** | **88** |
| **RBC** | **397** | **6** | **13** | **5** | **5** | **3** | **4** |
| **PLT** | **17.3** | **26** | **41.6** | **37.4** | **33.6** | **33.9** | **31.9** |

Example 2

This Example aims to confirm the state of precipitation of erythrocytes with or without an anticoagulant added at the time of adding PMSB represented by formula VII.
Five samples were prepared by the following method. As in Example 1, 1.35 mL of blood was added to the ACD solution to prepare a whole blood sample (control) (Sample 1) having a total of 1.5 mL. Various weights of PMSB were dissolved in the ACD solution or the Hanks' solution (supplied by Invitrogen), and then added to blood to prepare Samples of 2 to 5. Here, PMSB25 in Samples 2 and 4 is a PMSB (molecular weight of 100, 000) comprising MPC, PMPS and BMA described in formula VII in a ratio of 25:20:55, and PMSB45 in samples 3 and 5 is a PMSB (molecular weight of 100,000) comprising the compounds described in formula VII in a ratio of 45:5:50 likewise. Examples described below follow the same manner.
Sample 1: Control
Sample 2: 3 mg of PMSB25 was dissolved in 0.15 ml of the ACD solution, and then mixed with 1.35 mL of blood.
Sample 3: 3 mg of PMSB45 was dissolved in 0.15 ml of the ACD solution, and then mixed with 1.35 mL of blood.
Sample 4: 3 mg of PMSB25 was dissolved in 0.15 ml of the Hanks' solution, and then mixed with 1.35 mL of blood.
Sample 5: 3 mg of PMSB45 was dissolved in 0.15 ml of the Hanks' solution, and then mixed with 1.35 mL of blood.

Each sample described above was allowed to stand for 30 minutes. In Samples 2 and 3, erythrocytes were observed to precipitate, thereby to give a supernatant, from which platelet-rich plasma could be obtained. On the other hand, in Samples 4 and 5 of systems without an anticoagulant contained, the samples were coagulated, and platelet-rich plasma could not be obtained.

Example 3

This Example aims to examine effects of various PMSB added.
The following 4 samples were prepared as in the method of Examples 1 and 2.

Sample 1: Control
Sample 2: 3 mg of polyglutamic acid was dissolved in 0.15 ml of the ACD solution, and then mixed with 1.35 mL of blood.
Sample 3: 3 mg of PMSB25 was dissolved in 0.15 ml of the ACD solution, and then mixed with 1.35 mL of blood.
Sample 4: 3 mg of PMSB45 was dissolved in 0.15 ml of the ACD solution, and then mixed with 1.35 mL of blood.

Each sample described above was allowed to stand for 30 minutes. In samples 2 to 4, erythrocytes were observed to precipitate, thereby to give a supernatant, from which platelet-rich plasma was obtained. Leukocyte (WBC) and platelet (PLT) counts in the above described supernatant after standing, coagulation time and platelet activity (CD62P) were shown Table 2.
0.5 mL of supernatants of each sample, 0.05 mL of calcium chloride injection solution (as $CaCl_2$, 1.11 g/20 mL) in Pharmacopeia of Japan, and further 0.05 mL of self-blood with no anticoagulant were mixed in a 1.5 ml Eppendorf tube, and then the tube was slanted every 5 minutes to observe coagulation, and coagulation time was measured.
CD62P is a glycoprotein which is present in secreted granulosa in a platelet and has a molecular weight of 140kd, and once the platelet is activated, it moves to the surface of cell membrane of the platelet and expresses. Therefore, it serves as an indicator for activation of a platelet. CD62P was measured by flow cytometry supplied by BD Biosciences using an antibody.
As a result, times needed for platelet-rich plasmas obtained from Samples 3 and 4 to coagulate were from 25 to 50 minutes, indicating that they had excellent coagulation ability in comparison to the platelet-rich plasma obtained from Sample 2. Further, the platelet-rich plasma obtained from Sample 2 had a high CD62P value, indicating that the platelet had an enhanced activity, but the platelet-rich plasmas obtained from Samples 3 and 4 had low CD62P values (Table 2).

[Table 2]

| **Number of Blood Cells** | | | | |
|---|---|---|---|---|
| | **Sample 1** | **Sample 2** | **Sample 3** | **Sample 4** |
| **WBC** | **83** | **112** | **153** | **170** |
| **PLT** | **19.2** | **34.3** | **41.7** | **41.8** |
| **Coagulation Time** | | | | |
| | **Sample 1** | **Sample 2** | **Sample 3** | **Sample 4** |
| **Time** | — | **Coagulated after 90 minutes** | **50 min.** | **25 min.** |
| **CD62P (%Gated)** | | | | |
| | **Sample 1** | **Sample 2** | **Sample 3** | **Sample 4** |
| **%Gated** | *** | **95.73** | **4.58** | **4.42** |

Example 4

This Example aims to examine effects of various PMSB added.
The following four samples were prepared as in Example 3.

Sample 1: Control
Sample 2: 3 mg of polyglutamic acid was dissolved in 0.15 ml of the ACD solution, and then mixed with 1.35 mL of blood.
Sample 3: 3 mg of PMSB25 was dissolved in 0.15 ml of the ACD solution, and then mixed with 1.35 mL of blood.
Sample 4: 3 mg of PMSB45 was dissolved in 0.15 ml of the ACD solution, and then mixed with 1.35 mL of blood.

Each sample described above was allowed to stand for 30 minutes. In Samples 2 to 4, erythrocytes were observed to precipitate to give a supernatant, from which the platelet-rich plasma was obtained. Leukocyte (WBC) and platelet (PLT) counts in the above described supernatant after standing and coagulation time were measured according to the method described in Examples 1 and 3.
As a result, times needed for platelet-rich plasmas obtained from Samples 3 and 4 to coagulate were from 10 to 20 minutes, indicating that they had excellent coagulation ability in comparison to platelet-rich plasma obtained from Sample 2 (Table 3).

[Table 3]

| Number of Blood Cells | | | | |
|---|---|---|---|---|
| | **Sample 1** | **Sample 2** | **Sample 3** | **Sample 4** |
| **WBC** | **89** | **138** | **161** | **160** |

(continued)

| Number of Blood Cells | | | | |
|---|---|---|---|---|
| | **Sample 1** | **Sample 2** | **Sample 3** | **Sample 4** |
| **PLT** | **22.1** | **41.6** | **45** | **46.4** |
| Coagulation Time | | | | |
| | **Sample 1** | **Sample 2** | **Sample 3** | **Sample 4** |
| **Time** | — | 35min. | 20min. | 10min. |

Example 5

This Example aims to examine effects of various polymers added.

The following five samples were prepared. 3.13 w/v% ACD solution of ACD added in water and the control of Sample 1 were prepared as in Example 1. For Samples 3 and 4, a homopolymer composed of only MPC as a constituent unit (molecular weight of 100,000) as described in formula IV were used, while for Sample 5, a heteropolymer comprising MPC and BMA in a ratio of 8:2 (PMB (8:2)) (molecular weight of 100,000) as described in formula VI was used. Various polymers were dissolved in water to get 5 w/v% as their polymer solutions.

Sample 1: Control

Sample 2: 3mg of polyglutamic acid was dissolved in 0.15 ml of the ACD solution, and then mixed with 1.35 mL of blood.

Sample 3: 3mg of polyglutamic acid was added to 0.06 mL of polymer solution (PMPC), supplied with 0.09 ml of the ACD solution, and then mixed with 1.35 mL of blood.

Sample 4: 0.09 ml of the ACD solution was added to 0. 06 mL of polymer solution (PMPC), and then mixed with 1.35 mL of blood.

Sample 5: 0.09 ml of the ACD solution was added to 0. 06 mL of polymer solution (PMB (8:2)), and then mixed with 1.35 mL of blood.

Leukocyte (WBC) and platelet (PLT) counts in the supernatants after the samples described above were allowed to stand for 30 minutes, coagulation times and activated-platelet rates (CD62P) were measured as in Examples 1 and 3. As a result, it was confirmed that in the platelet-rich plasmas obtained from Samples 2 and 3 with polyglutamic acid contained, CD62P showed high values, while in the platelet-rich plasmas obtained from Samples 4 and 5, CD62P showed low values. Further, the platelet-rich plasmas obtained from Samples 4 and 5 coagulated within 10 minutes, while the platelet-rich plasmas obtained from Samples 2 and 3 with polyglutamic acid contained needed longer times for coagulation even the same amount of calcium chloride was added as in Samples 4 and 5. Further, the platelet-rich plasmas obtained from Samples 4 and 5, even if they were not supplied with a blood with no anticoagulant added, were supplied only with calcium chloride to coagulate within 10 minutes (Table 4).

[Table 4]

| **Number of Blood Cells** | | | | | |
|---|---|---|---|---|---|
| | **Sample 1** | **Sample 2** | **Sample 3** | **Sample 4** | **Sample 5** |
| **WBC** | **69** | **103** | **108** | **142** | **124** |
| **P LT** | **21.3** | **45.3** | **43.8** | **51.6** | **42.9** |
| Coagulation Time | | | | | |
| | **Sample 1** | **Sample 2** | **Sample 3** | **Sample 4** | **Sample 5** |
| | *** | **Not coagulated after 35 min. Afterwards coagulated.** | **10~35 min** | **Within 10 min.** (Coagulated only with $CaCl_2$) | **Within 10 min.** (Coagulated only with $CaCl_2$) |

(continued)

| CD62P (%Gated) | | | | | |
|---|---|---|---|---|---|
| | **Sample 1** | **Sample 2** | **Sample 3** | **Sample 4** | **Sample 5** |
| **After 30 min.** | *** | **97.55** | **97.77** | **9.97** | **3.82** |

Example 6

This Example aims to measure amount of fibrinogen contained in both platelet-rich plasmas obtained by the method of the present invention and PRP.

The following three samples were prepared. Samples 1 and 2 were allowed to stand for 30 minutes according to the method of Example 1. For Sample 3, blood was added to 0.85 mL of aqueous solution with ACD3.8 w/v% mixed to prepare a solution having a total of 8.5 mL with the use of PRPkit (Kurasan KK), which was centrifuged by 3600 rpm for 15 minutes and then further by 2400 rpm for 10 minutes to give a supernatant.

Sample 1: Control
Sample 2: 3mg of PMB (8:2) was dissolved in 0.15 mL of the ACD solution, and then mixed with 1.35 mL of blood.
Sample 3: Conventional method (centrifugation)

In the supernatants obtained from the samples described above, the amounts of fibrinogens were measured. Measurement was conducted by the thrombin method using reagent from Boehringer Mannheim.
As a result, in Samples 1 and 2, high fibrinogen values were obtained. From this fact, it was confirmed that the platelet-rich plasmas obtained by the method of the present invention was neither denatured nor lost during operation, and thus had a large amount of fibrinogen (Table 5).

[Table 5]

| | **Sample 1** | **Sample 2** | **Sample 3** |
|---|---|---|---|
| **Fibrinogen (mg/dl)** | **212** | **214** | **184** |

Example 7

This Example aims to examine coagulation time when a heteropolymer represented by Formula (V) is added. The following two samples were prepared. Sample 1 was allowed to stand for 20 and 30 minutes according to the method of Example 1 to give a supernatant. For Sample 2, 0.85 mL of 3.8 w/v% ACD in water was added to blood to give a solution having a total of 8.5 mL, which was then placed into a 15 ml conical tube (BD Falcon tube) and centrifuged by 1000 rpm for 10 minutes to get a supernatant. The state of precipitation of erythrocytes in Sample 1 was shown in Figure 1.

Sample 1: 3mg of both MPC and methacrylic acid copolymer (3:7) was dissolved in 0.15 mL of the ACD solution, and then mixed with 1.35 mL of blood.
Sample 2: Conventional method (centrifugation)

Coagulation times were measured of the supernatants obtained from the samples described above. As in Example 3, 0.5 mL of the supernatant of each sample, 0.05 mL of calcium chloride injection solution (as $CaCl_2$, 1.11 g/ 20 mL) in Pharmacopeia of Japan, and further 0.05 mL of self-blood with no anticoagulant added were mixed in a 1.5 mL Eppendorf tube, and then the tube was slanted every 1 minute from 5 minutes after mixing to observe coagulation. As a result, coagulation was observed in Sample 1 in 8 minutes, while Sample 2 obtained by a conventional method took 13 minutes to coagulate. From this fact, it was confirmed that the platelet-rich plasma obtained by the method of the present invention had excellent coagulation ability (Table 6).

[Table 6]

| | **Sample 1** | **Sample 2** |
|---|---|---|
| **Time to Coagulate** | **8 min.** | **13 min.** |

Example 8

This Example aims to examine platelet recovery rate, amount of fibrinogen and activated-platelet rate (CD62P) when PMB polymer is added.
The following two samples were prepared. Sample 1 was allowed to stand for 30 minutes according to the method of Example 5, yielding a supernatant. For sample 2, blood was added to 0.85 mL of aqueous solution with ACD3.8 w/v% mixed to prepare a solution having a total of 8.5 mL, which was placed in a 15 mL conical tube (BD Falcon tube) and centrifuged by 2400 rpm for 10 minutes, followed by collecting a supernatant with platelets contained and further centrifuging by 3600 rpm for 15 minutes to give a supernatant.
Sample 1: 0.09 mL of ACD solution was added to 0.06 mL of polymer solution (PMB (8:2)), and then mixed with 1.35 mL of blood.

Sample 2: Conventional method (centrifugation)

For supernatants obtained from the samples described above, platelet recovery rates, amount of fibrinogen and CD62P were measured.
Platelet recovery rates were obtained according to the Formula below.

$$\{(\text{amount of a supernatant} \times \text{platelet concentration in the supernatant}) / (\text{amount of blood collected} \times \text{platelet concentration at collecting blood})\} \times 100\ (\%)$$

CD62P was measured according to the method described in Example 3.
The amount of fibrinogen was measured according to the method described in Example 6.
There were shown platelet recovery rate of supernatant obtained from each sample in Figure 2, amount of fibrinogen in Figure 3, and activated-platelet rate (CD62P) in Figure 4. As a result, in platelet recovery rate and amount of fibrinogen, the platelet-rich plasma obtained from Sample 1 was equivalent to that obtained from Sample 2 (conventional method). Further, in activated-platelet rate, the platelet-rich plasma obtained from Sample 1 was suppressed to have a lower value, and thus it was confirmed that this platelet-rich plasma had more excellent performance than the supernatant obtained in sample 2.

[Industrial Applicability]

As explained above, platelet-rich plasma obtained by the preparation method of the present invention was comparable to PRP obtained by conventional centrifugation in platelet recovery rate and amount of fibrinogen and kept a very high platelet activity. Therefore, platelet-rich plasma with excellent performance can be provided conveniently. In particular, platelet-rich plasma obtained by the method of the present invention using a self-blood component as the source may be employed in the field of medical treatment as a tissue and/or organ repair enhancer, in particular, an augmentation additive of bone surrounding dental implant, an additive for implanting bone or artificial bone into a bone deficient part, a wound healing enhancer, a tissue healing enhancer after therapy or treatment for plastic and/or cosmetic purpose, a skin disease therapeutic agent, a skin ulcer therapeutic agent and a nerve tissue repair agent and/or a tissue repair agent after surgery.

## Claims

1. A method for preparing platelet-rich plasma, comprising adding a polymer having a residue of an organic phosphate compound to blood.

2. A method for preparing platelet-rich plasma, comprising the step of adding a polymer having a residue of an organic phosphate compound to blood to selectively precipitate erythrocytes.

**3.** The method for preparing platelet-rich plasma according to Claim 1 or 2, comprising, as the polymer having a residue of an organic phosphate compound, a compound having in its side chain a group represented by the Formula (I) below:

[Chem. 1]

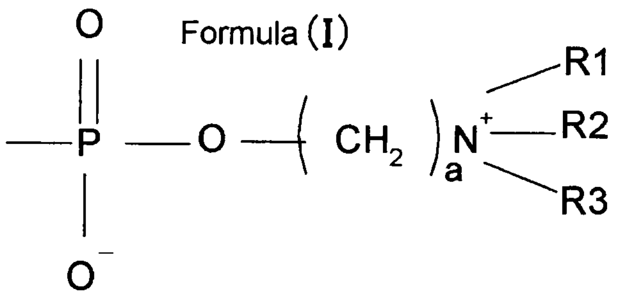

(wherein R1, R2 and R3 are each independently a straight or branched chain alkyl group having a carbon number of 1 to 8, and a is an integer of 1 to 12).

**4.** The method for preparing platelet-rich plasma according to Claim 3, comprising, as the polymer having a residue of an organic phosphate compound, a compound represented by the Formula (II) below:

[Chem. 2]

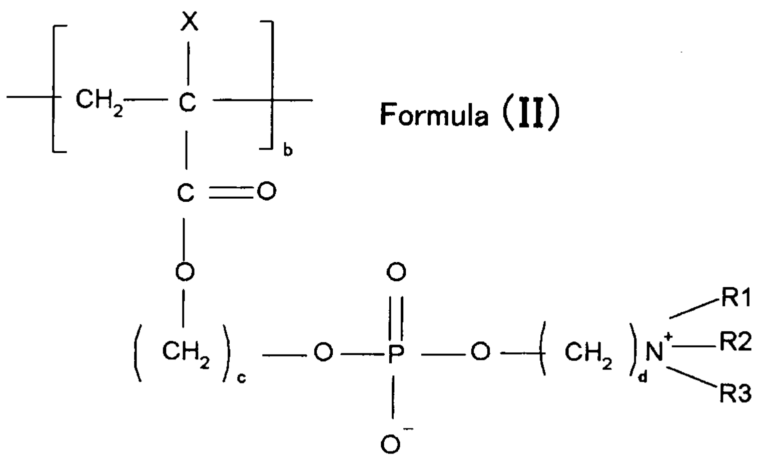

(wherein X is -H or $-CH_3$, R1, R2 and R3 are each independently a straight or branched chain alkyl group having a carbon number of 1 to 8, b is an integer of 1 to 4,000, c is an integer of 1 to 6, and d is an integer of 1 to 12).

**5.** The method for preparing platelet-rich plasma according to any one of Claims 1 to 4, further comprising, as the polymer having a residue of an organic phosphate compound, a compound represented by the Formula (III) below:

[Chem. 3]

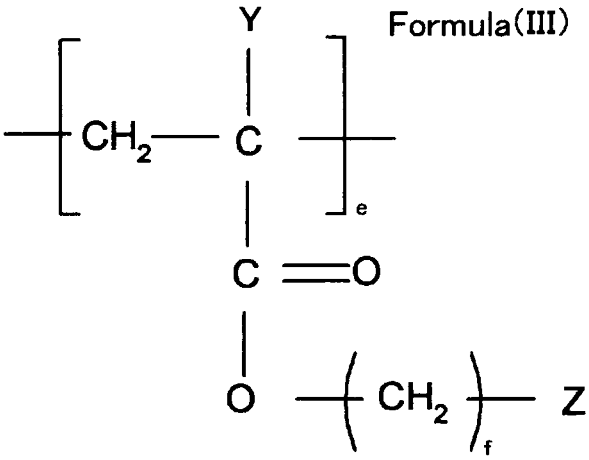
Formula (III)

(wherein Y is -H or $-CH_3$, Z is -H, -OH, -COOH, -COONa, -COOK, $-SO_3H$, $-SO_3Na$, $-SO_3K$, $-NH_2$, -NHR1, -NR1R2, $-N^+R1R2R3OH^-$, $-N^+R1R2R3SO_3$-, $-N^+R1R2R3NO_3$-, $-N^+R1R2R3PO_3$- [wherein R1, R2 and R3 are independently $-(CH_2)xH$, wherein x is an integer of 1 to 5], $-O-PO_3Na$, $-O-PO_3K$, $-O-PO_3H$, -OR4H, -COOR4H, -C=O-R4H, [wherein R4 is aliphatic hydrocarbon, wherein the number of hydrocarbon is 0 to 20] or -O-R5 [R5 is an aromatic hydrocarbon group], e is an integer of 1 to 4,000, and f is an integer of 0 to 20).

**6.** The method for preparing the platelet-rich plasma according to Claim 4 or 5, comprising, as the polymer having a residue of an organic phosphate compound, a compound represented by the Formula (IV) below:

[Chem. 4]

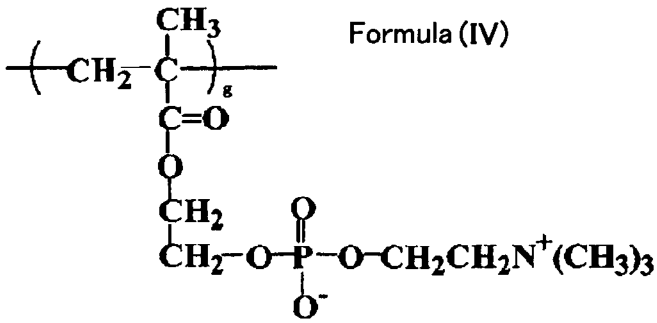
Formula (IV)

(wherein g is an integer of 1 to 4,000).

**7.** The method for preparing the platelet-rich plasma according to any one of Claims 3 to 6, comprising, as the polymer having a residue of an organic phosphate compound, a compound represented by any of the Formulae (V) to (VII) below:

[Chem. 5]

Formula (V)

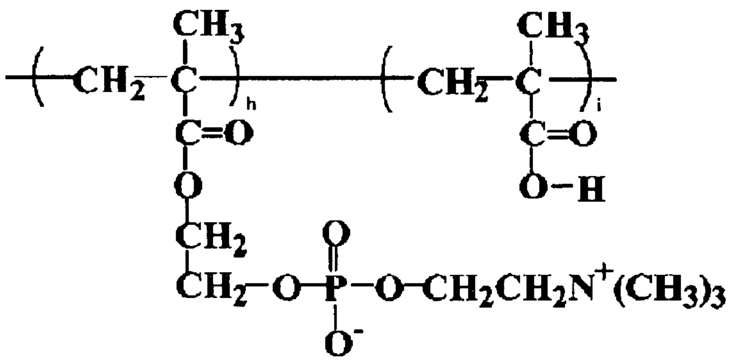

(wherein h and i are each independently an integer of 1 to 4,000),

[Chem. 6]

Formula (VI)

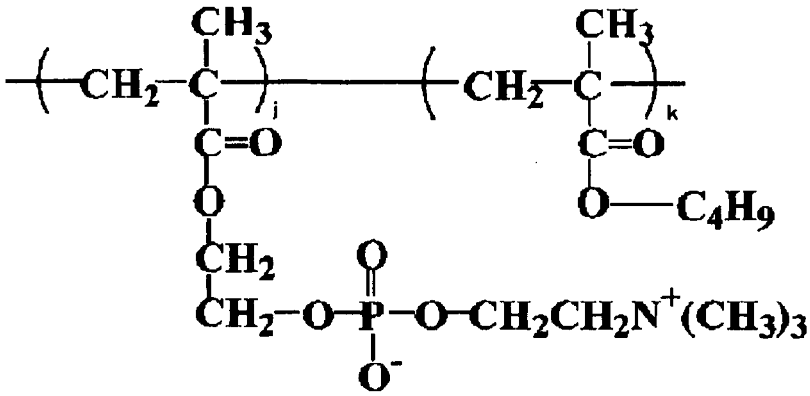

(wherein j and k are each independently an integer of 1 to 4,000), and

[Chem. 7]

Fromula (VII)

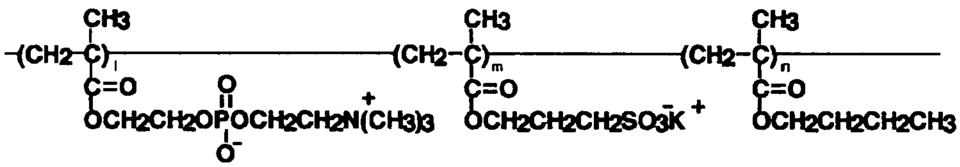

(wherein 1, m and n are each independently an integer of 1 to 4,000).

**8.** A platelet-rich plasma obtained by the method for preparing according to any one of Claims 1 to 7.

**9.** A tissue and/or an organ repair enhancer comprising the platelet-rich plasma according to Claims 8.

**10.** A tissue and/or an organ repair enhancer, augmentation additive of bone surrounding dental implant, additive when implanting bone or artificial bone into a bone deficient part, wound healing enhancer, tissue healing enhancer after therapy or treatment for plastic and/or cosmetic purpose, skin disease therapeutic agent, skin ulcer therapeutic agent, nerve tissue repair agent and/or tissue repair agent after surgery comprising the platelet-rich plasma according to Claim 8.

**11.** Any of the following therapeutic methods or treatment methods, comprising administering the platelet-rich plasma according to Claim 8:

1) augmenting bone surrounding dental implant,
2) skin disease,
3) repairing tissue for plastic and/or cosmetic purpose,
4) repairing bone deficient part,
5) repairing nerve tissue, and
6) repairing tissue after surgery.

**12.** A polymer for preparing the platelet-rich plasma according to Claim 8.

**13.** A reagent or a reagent kit for preparing platelet-rich plasma comprising the polymer according to Claim 12.

**14.** A device for preparing the platelet-rich plasma according to Claim 8.

Fig. 1

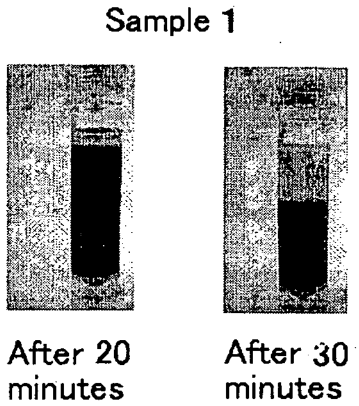
Sample 1
After 20 minutes
After 30 minutes

Fig.2

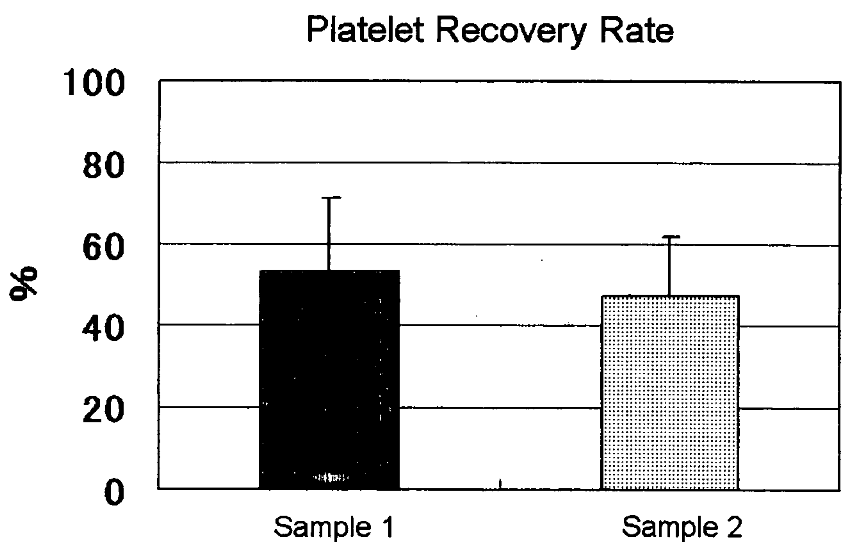
Platelet Recovery Rate
100
80
60
%
40
20
0
Sample 1
Sample 2

Fig.3

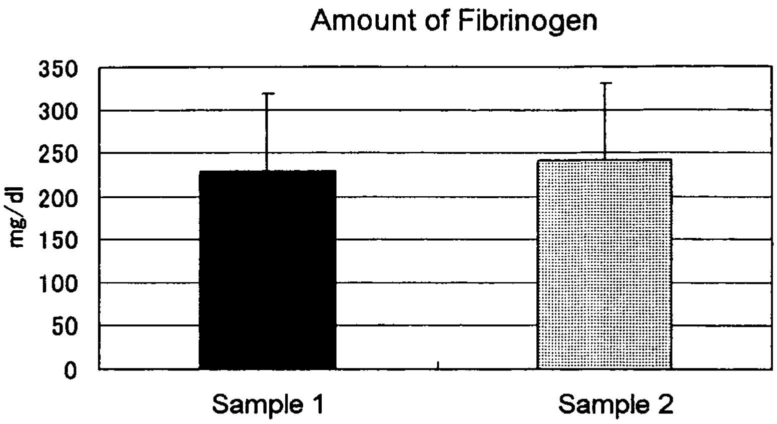
Amount of Fibrinogen
350
300
250
200
150
100
50
0
mg/dl
Sample 1
Sample 2

Fig.4

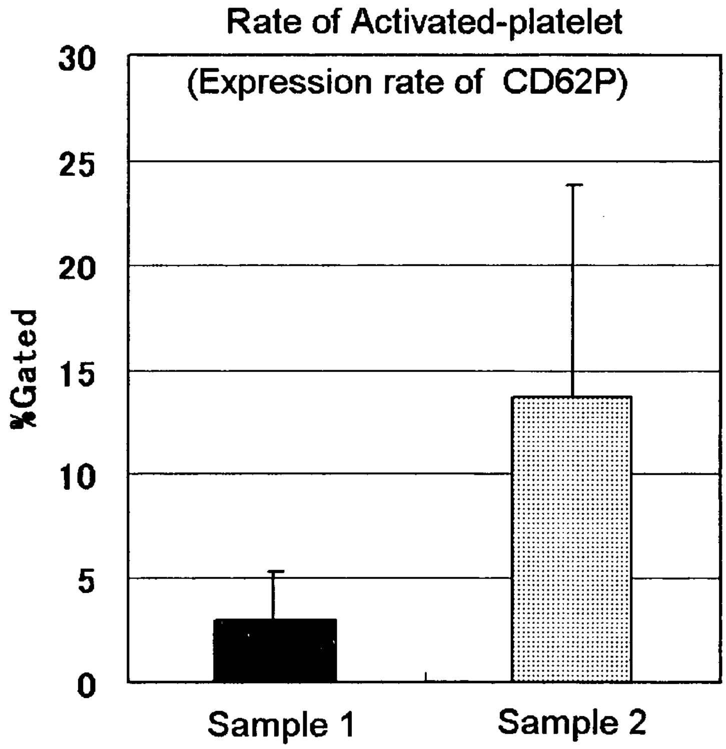
Rate of Activated-platelet
(Expression rate of CD62P)
30
25
20
15
10
5
0
%Gated
Sample 1
Sample 2

**INTERNATIONAL SEARCH REPORT**

International application No.
PCT/JP2005/001231

A. CLASSIFICATION OF SUBJECT MATTER
Int.Cl[7] A61K35/16, A61L27/00, A61P1/02, A61P17/02, A61P19/00, A61P25/00, A61P41/00, A61P43/00, A61J1/00

According to International Patent Classification (IPC) or to both national classification and IPC

B. FIELDS SEARCHED

Minimum documentation searched (classification system followed by classification symbols)
Int.Cl[7] A61K35/14-35/16

Documentation searched other than minimum documentation to the extent that such documents are included in the fields searched

Electronic data base consulted during the international search (name of data base and, where practicable, search terms used)
CAPlus(STN), BIOSIS(STN), MEDLINE(STN), EMBASE(STN), WPIDS(STN), JOIS

C. DOCUMENTS CONSIDERED TO BE RELEVANT

| Category* | Citation of document, with indication, where appropriate, of the relevant passages | Relevant to claim No. |
|---|---|---|
| X<br>Y | JP 2002-98676 A (Kazuhiko ISHIHARA),<br>05 April, 2002 (05.04.02),<br>Full text; Claim 9; Par. Nos. [0002], [0005] to [0016], [0017], [0022] to [0025], [0031] to [0032], [0036] to [0039]<br>(Family: none) | 1-8,12-14<br>9,10 |
| X<br>Y | JP 8-510322 A (INTERNATIONAL REMOTE IMAGING SYSTEMS INC.),<br>29 October, 1996 (29.10.96),<br>Full text<br>& WO 94/25135 A1 & AU 9466358 B<br>& US 5397479 A & US 5482829 A<br>& EP 696933 A1 | 8,14<br>9,10 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 06 April, 2005 (06.04.05) | 24 May, 2005 (24.05.05) |

| Name and mailing address of the ISA/ | Authorized officer |
|---|---|
| Japanese Patent Office | |
| Facsimile No. | Telephone No. |

Form PCT/ISA/210 (second sheet) (January 2004)

**INTERNATIONAL SEARCH REPORT**

International application No. PCT/JP2005/001231

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | JP 10-296063 A (NOF Corp.),<br>10 November, 1998 (10.11.98),<br>Full text; Claims; Par. Nos. [0001], [0034]; examples<br>(Family: none) | 12<br>1-10,13,14 |
| X<br>Y | JP 2000-212376 A (NOF Corp.),<br>02 August, 2000 (02.08.00),<br>Full text; Claims; Par. No. [0048]; examples<br>(Family: none) | 12<br>1-10,13,14 |
| X<br>Y | Hideyuki KONO et al., 'Shushu no Suiyosei Polymer o Graft-Ka shita Cellulose Ketsueki Tosekimaku Hyomen no Ketsueki Tekigosei', Biodegradable Gel, (1998), Vol.55, No.6, pages 334 to 343; Full text; abstract | 12<br>1-10,13,14 |
| X<br>Y | Yasuhiko IWASAKI et al., 'Phospholipid Kyokuseiki o Yusuru Polymer no ex vivo ni Okeru Ketsueki Tekigosei no Hyoka', Journal of Japanese Society for Biomaterials, (1995), Vol.13, No.2, pages 62 to 70; Full text | 12<br>1-10,13,14 |
| A | ISHIHARA, K. et al., 'STABILIZED LIPOSOMES WITH PHOSPHOLIPID POLYMERS AND THEIR INTERACTIONS WITH BLOOD CELLS.', COLLOIDS AND SURFACES B: BIOINTERFACES, (2002), Vol.25, No.4, pages 325 to 333; Full text | 1-10,12-14 |
| A | JP 60-231613 A (Terumo Corp.),<br>18 November, 1985 (18.11.85),<br>Full text; Claims; page 1, lower right column, lines 9 to 11; page 4, lower right column, line 5 to page 5, upper left column, line 11<br>& EP 163146 A1 & US 4836928 A | 1-10,12-14 |
| A | JP 56-128718 A (Asahi Chemical Industry Co., Ltd.),<br>08 October, 1981 (08.10.81),<br>Full text; Claims; page 1, lower left column, 3rd line from the bottom to page 2, upper left column, line 15; page 3, upper right column, line 8 to lower right column, 2nd line from the bottom; examples<br>& FR 2477882 A1 & JP 56-128717 A<br>& GB 2077137 A & DE 3109493 A1<br>& US 4370381 A & US 4476023 A | 1-10,12-14 |
| A | JP 5-148151 A (Asahi Medical Co., Ltd.),<br>15 June, 1993 (15.06.93),<br>Full text; Claims; Par. Nos. [0009] to [0013], [0020] to [0021], [0023], [0025]; example 1<br>(Family: none) | 1-10,12-14 |

Form PCT/ISA/210 (continuation of second sheet) (January 2004)

**INTERNATIONAL SEARCH REPORT**

International application No.
PCT/JP2005/001231

C (Continuation). DOCUMENTS CONSIDERED TO BE RELEVANT

| Category* | Citation of document, with indication, where appropriate, of the relevant passages | Relevant to claim No. |
|---|---|---|
| A | JP 2002-267677 A (Jun KIKUCHI),<br>18 September, 2002 (18.09.02),<br>Full text; Claims; 16, 17; Par. No. [0013]<br>(Family: none) | 1-10,12-14 |
| Y | JP 2003-55237 A (Japan Science and Technology Corp.),<br>26 February, 2003 (26.02.03),<br>Full text<br>(Family: none) | 9,10 |
| P,A | WO 04/12750 A1 (SUMIDA E),<br>12 February, 2004 (12.02.04),<br>Full text<br>& AU 2003/252316 A1 | 1-10,12-14 |

Form PCT/ISA/210 (continuation of second sheet) (January 2004)

**INTERNATIONAL SEARCH REPORT**

International application No.
PCT/JP2005/001231

**Box No. II Observations where certain claims were found unsearchable (Continuation of item 2 of first sheet)**

This international search report has not been established in respect of certain claims under Article 17(2)(a) for the following reasons:

1. [X] Claims Nos.: 11
because they relate to subject matter not required to be searched by this Authority, namely:
Claim 11 pertains to methods for treatment of the human body by therapy and thus relates to a subject matter which this International Searching Authority is not required, under the provisions of Article 17(2)(a)(i) of the PCT and Rule 39.1(iv) of the Regulations under the PCT, to search.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

(See extra sheet.)

1. [ ] As all required additional search fees were timely paid by the applicant, this international search report covers all searchable claims.
2. [X] As all searchable claims could be searched without effort justifying an additional fee, this Authority did not invite payment of any additional fee.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**
[ ] The additional search fees were accompanied by the applicant's protest.
[ ] No protest accompanied the payment of additional search fees.

Form PCT/ISA/210 (continuation of first sheet (2)) (January 2004)

**INTERNATIONAL SEARCH REPORT**

International application No.
PCT/JP2005/001231

Continuation of Box No.III of continuation of first sheet(2)

<Unity of invention>

[1] Claims 1 to 7 (referred to as invention group 1)
[2] Claims 8 to 10 (referred to as invention group 2)
[3] Claims 12 and 13 (referred to as invention group 3)
[4] Claim 14 (referred to as invention group 4)

Although the platelet-rich plasma as defined in the invention group 2 depends on the preparation method according to the invention group 1, it is recognized that, from the components of the composition, the platelet-rich plasma involves an embodiment relating to a platelet-rich plasma fraction having been known by a person skilled in the art before the priority date of the present case as described in the following document:

JP 8-510322 A (INTERNATIONAL REMOTE IMAGING SYSTEM INC.)
29 October, 1995 (29.10.95), full text
(the document cited in column C in the present international search report)

Thus, the platelet-rich plasma which is an invention-specifying matter common to the invention groups 1 and 2 cannot be considered as a special technical feature.

Also, a polymer having an organophosphoric acid compound per se, which pertains to the definition in any of claims 1 to 7 of the invention group 1 and pertains to the polymer of the invention group 3 too, had been well known as a chemical before the priority date of the present case as described in, for example, the following document:

JP 2002-98676 A (Kazuhiko ISHIHARA)
05 April, 2002 (05.04.02), full text, Claim 9, Par. Nos. [0002], [0005] to [0016], [0017], [0022] to [0025], [0031] to [0032], [0036] to [0039]
(the document cited in column C in the present international search report)

Moreover, it is not defined that the polymer as specified by the invention group 3 has a chemical structure common to an arbitrary polymer as specified by the invention group 1 and, therefore, there is no special technical feature common to the invention groups 1 and 3.

The instrument according to the invention group 4 is restricted to those aiming at preparing the platelet-rich plasma as claimed in claim 8. However, the platelet-rich plasma per se is not characteristic as discussed above and instruments per se (for example, a syringe, a blood-collection tube, a plastic container, a test tube and so on) had been commonly known by a person skilled in the art before the priority date of the present case. Thus, it does not appear that there is a special technical feature between the invention group 4 and any of the invention groups 1 to 3.

Such being the case, it does not appear that there are the same or corresponding special technical features between any two of the invention groups 1 to 4 as described above and, therefore, these four groups of invention are not considered as being so linked as to form a single general inventive concept.

Form PCT/ISA/210 (extra sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2004024815 A
- JP H5203639 A
- JP H8510322 B


### Non-patent literature cited in the description


- Outline of Clinical Laboratory Testing. 400